# EUROPEAN PATENT SPECIFICATION

(11) **EP 4 197 537 B1**
(45) Date of publication and mention of the grant of the patent: **23.07.2025**
(21) Application number: 21852513.7
(22) Date of filing: 04.08.2021
(51) Int. Cl.: A61K 31/506, A61P 1/16, A23L 33/10, G01N 33/50

(54) **COMPOSITION FOR PREVENTING OR TREATING LIVER FIBROSIS, CONTAINING TRIAZOLE DERIVATIVE AS ACTIVE INGREDIENT**
ZUSAMMENSETZUNG ZUR PRÄVENTION ODER BEHANDLUNG VON LEBERFIBROSE MIT TRIAZOLDERIVAT ALS WIRKSTOFF
COMPOSITION POUR PRÉVENIR OU TRAITER LA FIBROSE HÉPATIQUE, CONTENANT UN DÉRIVÉ DE TRIAZOLE COMME PRINCIPE ACTIF

(30) Priority: 04.08.2020 KR 20200097358
(43) Date of publication of application: 21.06.2023
(73) Proprietor: Yonsei University BioHealth Technology Holdings, Inc., Seoul 03722 (KR)
(72) Inventor: CHUN, Kyung-Hee, Seoul 03180 (KR); BAEK, Jung-Hwan, Seoul 03743 (KR)
(74) Representative: Müller, Christian Stefan Gerd
(86) International application number: PCT/KR2021/010189
(87) International publication number: WO 2022/030976

(56) References cited:
- WO-A1-02/22584
- WO-A1-2020/005935
- WO-A2-2014/105952
- JP-A- H05 255 327
- US-A1- 2019 151 318
- LIAO YUNING ET AL: "USP1-dependent RPS16 protein stability drives growth and metastasis of human hepatocellular carcinoma cells", vol. 40, no. 1, 21 June 2021 (2021-06-21), London UK, XP093180947, ISSN: 1756-9966, Retrieved from the Internet <URL:https://link.springer.com/article/10.1186/s13046-021-02008-3/fulltext.html> DOI: 10.1186/s13046-021-02008-3
- ANONYMOUS YALEI ET AL: "Cell Proliferation | Cell Biology Journal | Wiley Online Library", vol. 53, no. 10, 1 October 2020 (2020-10-01), GB, XP093181050, ISSN: 0960-7722, Retrieved from the Internet <URL:https://onlinelibrary.wiley.com/doi/10.1111/cpr.12908> DOI: 10.1111/cpr.12908
- KIM, MOON YOUNG: "The Diagnosis of Nonalcoholic Fatty Liver Disease", KOREAN JOURNAL OF MEDICINE, vol. 86, no. 4, 1 April 2014 (2014-04-01), pages 405 - 415, XP009533831, ISSN: 1738-9364, DOI: 10.3904/kjm.2014.86.4.405

## Description

### Technical Field

The present invention relates to a composition for use in a method of inhibiting liver tissue inflammation, fibrosis and the resulting hardening of liver tissue using a triazole derivative compound.

### Background Art

Chronic liver disease, one of adult diseases that account for the leading causes of death in Korea, is a serious disease that leads to liver cancer through various complications that determine the prognosis of liver disease after symptoms persist and progress to cirrhosis. Preventing the progression of chronic hepatitis to cirrhosis is one of the ultimate goals for treating liver diseases caused by various factors, such as dyslipidemia, infection with hepatitis B and C viruses, and the like. In addition, since cirrhosis is caused through the process of hepatic fibrosis, which is sustained by various causes, efficiently blocking and inhibiting the progress of hepatic fibrosis is the most basic and important step in preventing severe liver diseases such as liver cancer.

Hepatic fibrosis is caused by excessive deposition of extracellular matrix (ECM) in liver tissue due to chronic intrahepatic inflammation, and if this excessive deposition of ECM persists, it eventually progresses to cirrhosis accompanied by structural deformation of the liver and a reduction in the number of hepatocytes. Although studies on a therapeutic agent that effectively blocks this process from steatohepatitis through hepatic fibrosis to cirrhosis have been actively conducted, a therapeutic candidate substance, which effectively alleviates fibrosis and has safety suitable for long-term administration, has not been developed.

For example, thiazolidinedione (TZD), a PPAR-γ agonist, has been most extensively studied as a drug that treats steatohepatitis by promoting the secretion of adiponectin and improving insulin resistance in adipose, muscle and liver tissues. However, when thiazolidinedione (TZD) was administered, significant amelioration of fibrosis was not observed, and after discontinuation of administration, most cases returned to the pre-treatment state. In addition, thiazolidinedione (TZD) were reported to have side effects such as weight gain, bone loss, and increased incidence of bladder cancer, and thus the development thereof as a therapeutic agent is currently discontinued. Metformin, developed as a biguanide-based oral anti-diabetic agent, is an AMPK activator and was expected to have an effect against steatohepatitis and hepatic fibrosis, but in clinical studies for adult and pediatric patients, the histological improvement effect of metformin on steatohepatitis was not observed. In addition, statin, a therapeutic agent for hyperlipidemia, has no clear effect on non-alcoholic fatty liver, and some studies showed that statin reduced ALT levels and co-administration of statin and vitamins C and E reduced steatosis radiographically. However, statin was not effective in ameliorating fatty liver, inflammatory activity, and fibrosis in patients with steatohepatitis.

Accordingly, there has been an increasing demand for the development of a highly safe therapeutic agent which is suitable for long-term administration for the treatment of chronic diseases while efficiently blocking inflammation and fibrosis in liver tissue. Document US2019/151318 discloses how the disorders alcoholic hepatitis or alcoholic steatohepatitis (ASH) can be caused by an impaired ubiquitin-proteasome pathway function.

Through the specification, a number of publications and patent documents are referred to and cited.

### DISCLOSURE

### Technical Problem

The present inventors have made extensive research efforts to develop a pharmaceutical composition that effectively blocks the progress of a series of pathological stages leading to inflammation, fibrosis, and tissue hardening in liver tissue. As a result, the present inventors have found that, when a compound of Formula 1 is administered to a subject, the progression of fibrosis is significantly inhibited while the expression of inflammatory factors and collagen in liver tissue is significantly reduced, thereby completing the present invention.

Therefore, an object of the present invention is to provide a composition or a functional food composition for preventing or treating a liver disease selected from the group consisting of steatohepatitis, hepatic fibrosis and cirrhosis.

Another object of the present invention is to provide a in vitro method for screening a composition for inhibiting hepatic fibrosis.

Other objects and advantages of the present invention will become more apparent from the following detailed description of the invention, the appended claims and the accompanying drawings.

### Technical Solution

In accordance with one aspect of the present invention, the present invention provides a composition for preventing or treating a liver disease selected from the group consisting of steatohepatitis, hepatic fibrosis and cirrhosis, comprising a compound represented by the following Formula 1 or a pharmaceutically acceptable salt thereof as an active ingredient:

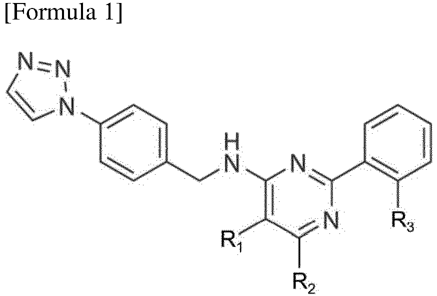

wherein R₁ and R₂ are each independently hydrogen or C₁-C₃ alkyl, R₁ and R₂ are not simultaneously hydrogen, and R₃ is C₂-C₄ alkyl.

The present inventors have made extensive research efforts to develop a pharmaceutical composition that effectively blocks the progress of a series of pathological stages leading to inflammation, fibrosis, and tissue hardening in liver tissue. As a result, the present inventors have found that, when the compound of Formula 1 is administered to a subject, the progression of fibrosis is significantly inhibited while the expression of inflammatory factors and collagen in liver tissue is significantly reduced.

As used herein, the term "alkyl" refers to a straight-chain or branched saturated hydrocarbon group, the term "C₁-C₃ alkyl" refers to an alkyl group having an alkyl unit having 1 to 3 carbon atoms, and when the C₁-C₃ alkyl is substituted, the carbon atom of the substituent is not included.

According to a specific embodiment of the present invention, R₁ is C₁ alkyl, R₂ is hydrogen, and R₃ is C₃ alkyl. More specifically, R₃ is an isopropyl group.

The compound of Formula 1, wherein R₁ is C₁ alkyl (methyl), R₂ is hydrogen, and R₃ is an isopropyl group, is a compound having the IUPAC name "N-(4-(1H-1,2,3-triazol-1-yl)benzyl)-2-(2-isopropylphenyl)-5-methylpyrimidin-4-amine" (CAS number: 1572414-83-5). The compound is known to have ubiquitin specific protease 1 (USP1) inhibitor activity, and thus has been suggested to have a therapeutic effect against USP1-mediated diseases such as breast cancer and ovarian cancer, but its relationship with hepatic fibrosis has not been known at all.

As used herein, the term "pharmaceutically acceptable salt" refers to a salt derived from a pharmaceutically acceptable inorganic acid, organic acid, or base. Examples of suitable acids include hydrochloric acid, hydrobromic acid, sulfuric acid, nitric acid, perchloric acid, fumaric acid, maleic acid, phosphoric acid, glycolic acid, lactic acid, salicylic acid, succinic acid, toluene-p-sulfonic acid, tartaric acid, acetic acid, trifluroacetic acid, citric acid, methanesulfonic acid, formic acid, benzoic acid, malonic acid, naphthalene-2-sulfonic acid, benzenesulfonic acid, and the like. Salts derived from suitable bases include salts of alkali metals such as sodium, alkaline earth metals such as magnesium, ammonium, and the like. Specifically, the pharmaceutically acceptable salt that is used in the present invention is a sodium salt.

As used herein, the term "steatohepatitis" refers to chronic inflammation in liver tissue caused by intrahepatic fat accumulation. Steatohepatitis is a pathological condition that is distinct from hepatic steatosis in which an abnormal level of fat is simply maintained in liver tissue or hepatocytes for a long time. Symptoms of steatohepatitis cannot be alleviated or eliminated by simply reducing lipids, and the fundamental treatment of steatohepatitis is possible only by the control of activated inflammatory cytokines and reactive oxygen species.

As used herein, the term "hepatic fibrosis" refers to a disease in which excessive fibrous connective tissue is formed in liver tissue by excessive deposition of the extracellular matrix due to chronic inflammation in the liver, resulting in damage to the normal structure and function of the liver.

As used herein, the term "cirrhosis" refers to a pathological condition in which normal liver tissue is lost as inflammation and extracellular matrix deposition in the liver tissue continue for a long time, and liver tissue does not function normally as scar tissue replaces the lost liver tissue.

Hepatic fibrosis and cirrhosis are not independent diseases with separate etiologies, but are continuous diseases progressing from chronic hepatitis through fibrosis to cirrhosis. To date, various therapeutic agents for metabolic diseases that have been commercially available because of their demonstrated lipid-lowering effect have failed to effectively alleviate hepatic fibrosis and hardening of liver tissue.

As used herein, the term "prevention" means inhibiting the occurrence of a disorder or a disease in a subject who has never been diagnosed as having the disorder or disease, but is likely to suffer from such disorder or disease.

As used herein, the term "treatment" means (a) inhibiting the progress of a disorder, disease or symptom; (b) alleviating the disorder, disease or symptom; or (c) eliminating the disorder, disease or symptom. When the composition of the present invention is administered to a subject, it functions to inhibit the progress of symptoms, or to eliminate or alleviate symptoms by reducing the production of excessive fibrous tissue in the skin by inhibiting the proliferation of skin fibroblasts and the expression of fibrogenic factors. Thus, the composition of the present invention may serve as a therapeutic composition for the disease alone, or may be administered in combination with other pharmacological ingredients and applied as a therapeutic aid for the disease. Accordingly, as herein used, the term "treatment" or "therapeutic agent" encompasses "treatment aid" or "therapeutic aid agent".

As used herein, the term "administration" or "administering" means administering a therapeutically effective amount of the composition of the present invention directly to a subject so that the same amount is formed in the subject's body.

As used herein, the term "therapeutically effective amount" refers to an amount of the composition containing a pharmacological ingredient (e.g., ethyl pyruvate) sufficient to provide a therapeutic or prophylactic effect to a subject to whom the pharmaceutical composition of the present invention is to be administered. Accordingly, the term "therapeutically effective amount" encompasses a "prophylactically effective amount".

As used herein, the term "administration" or "administering" means administering an effective amount of the composition of the present invention directly to a subject so that the same amount is formed in the subject's body.

As used herein, the term "subject" includes, without limitation, humans, mice, rats, guinea pigs, dogs, cats, horses, cows, pigs, monkeys, chimpanzees, baboons or rhesus monkeys. Specifically, the subject of the present invention is a human.

According to a specific embodiment of the present invention, steatohepatitis to be prevented or treated with the composition of the present invention is non-alcoholic steatohepatitis.

According to a specific embodiment of the present invention, the composition of the present invention reduces the level of ALT (alanine aminotransferase) or AST (aspartate aminotransferase) in the blood. ALT and AST are aminotransferases present in hepatocytes, and when hepatocytes are destroyed, ALT and AST are released into the blood and the levels thereof in the bloods increase. Thus, ALT and AST are used as liver function indicators. According to the present invention, the composition of the present invention significantly reduces the levels of AST and ALT in the blood by 55% and 30%, respectively, compared to the control group, thereby significantly restoring liver function reduced by steatohepatitis and hepatic fibrosis.

According to a specific embodiment of the present invention, the composition of the present invention reduces the expression of C-C motif chemokine ligand 2 (CCL2), transforming growth factor-β (TGF-β) and F4/80 in liver tissue.

As shown in Examples below, it was confirmed that the composition of the present invention significantly inhibits the expression of CCL2, TGF-β and F4/80, which are major inflammatory factors, indicating that the composition not only exhibits a simple and quantitative effect of reducing accumulated lipids, but also efficiently blocks the progress of inflammation and the resulting tissue damage.

As used herein, the term "reduced expression" means that the expression level of a protein or gene that is an indicator or cause of inflammation decreases to the extent that pathological inflammation and fibrosis in liver tissue are stopped, alleviated, or ameliorated, or the risk thereof is reduced. Specifically, it may mean a state in which the expression level has decreased by at least 20%, more specifically at least 30%, more specifically at least 40%, compared to the control group.

When the composition of the present invention is prepared as a pharmaceutical composition, the pharmaceutical composition of the present invention contains a pharmaceutically acceptable carrier.

Examples of the pharmaceutically acceptable carrier that is contained in the pharmaceutical composition of the present invention include, but are not limited to, lactose, dextrose, sucrose, sorbitol, mannitol, starch, gum acacia, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate, and mineral oil, which are commonly used in formulation. The pharmaceutical composition of the present invention may further contain a lubricant, a wetting agent, a sweetening agent, a flavoring agent, an emulsifying agent, a suspending agent, a preservative, and the like, in addition to the above-described components. Suitable pharmaceutically acceptable carriers and agents are described in detail in Remington's Pharmaceutical Sciences (19th ed., 1995).

The pharmaceutical composition of the present invention may be administered orally or parenterally. Specifically, it may be administered parenterally. More specifically, it may be administered subcutaneously or transdermally.

An appropriate dosage of the pharmaceutical composition of the present invention may vary depending on various factors such as formulation method, administration mode, patient's age, weight, sex, pathological condition, diet, administration time, administration route, excretion rate, and reaction sensitivity. A preferred dosage of the pharmaceutical composition of the present invention is within the range of 0.001 to 100 mg/kg for an adult.

The pharmaceutical composition of the present invention may be prepared in a unit dose form or prepared to be contained in a multi-dose container by formulating with a pharmaceutically acceptable carrier and/or excipient, according to a method that may be easily carried out by a person skilled in the art. Here, the formulation of the pharmaceutical composition may be a solution, suspension, syrup or emulsion of the pharmaceutical composition in oil or aqueous medium, or an extract, powder, granule, tablet or capsule containing the pharmaceutical composition, and may further contain a dispersing agent or a stabilizer.

In accordance with another aspect of the present invention, the present invention provides a functional food composition for treating a liver disease selected from the group consisting of steatohepatitis, hepatic fibrosis and cirrhosis, comprising a compound represented by the following Formula 1 or a food-acceptable salt thereof as an active ingredient:

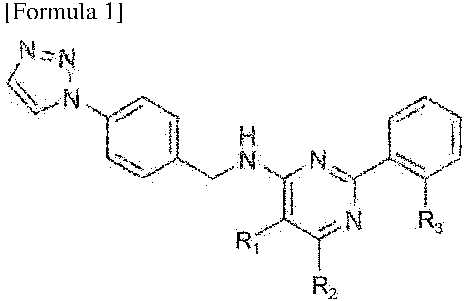

wherein R₁ and R₂ are each independently hydrogen or C₁-C₃ alkyl, R₁ and R₂ are not simultaneously hydrogen, and R₃ is C₂-C₄ alkyl.

Since the compound of Formula 1 used in the present invention and the liver disease to be ameliorated using the compound have already been described above, description thereof will be omitted to avoid excessive overlapping.

As used herein, the term "food-acceptable salt" refers to a salt in a form that may be used in a food composition, among salts composed of cations and anions bound together by electrostatic attraction, and specific examples thereof include examples of the above-described "pharmaceutically acceptable salts".

When the composition of the present invention is prepared as a food composition, it may contain not only the compound of the present invention as an active ingredient, but also carbohydrates, seasonings, and flavoring agents, which are commonly added in food preparation. Examples of the carbohydrates include, but are not limited to, monosaccharides such as glucose and fructose, disaccharides such as maltose and sucrose, polysaccharides such as dextrins and cyclodextrins, and sugar alcohols such as xylitol, sorbitol, and erythritol. Examples of the flavoring agents include natural flavoring agents [thaumatin, stevia extract (e.g., rebaudioside A, glycyrrhizin, etc.)] and synthetic flavoring agents (saccharin, aspartame, etc.). For example, when the food composition of the present invention is prepared as a drink, it may further contain, in addition to a pine bark extract as an active ingredient of the present invention, citric acid, liquid fructose, sugar, glucose, acetic acid, malic acid, fruit juice, eucommia extract, jujube extract, licorice extract, or the like.

In accordance with still another aspect of the present invention, the present invention provides a method for screening a composition for inhibiting hepatic fibrosis, the method comprising steps of:
(1) bringing a test substance into contact with a biological sample containing cells expressing USP1 (ubiquitin specific protease 1); and
(2) measuring the activity or expression level of USP1 in the sample,
wherein, when the activity or expression level of USP1 decreased, the test substance is determined as a composition for inhibiting hepatic fibrosis.

The correlation between fibrotic diseases triggered by inflammation, such as hepatic fibrosis, and the USP1 enzyme, is not yet known. According to the present invention, the present inventors have demonstrated for the first time that the USP1 enzyme can be a therapeutic target for hepatic fibrosis, by observing that the compound of the present invention, which has been reported to have USP1 inhibitory activity, inhibits hepatic fibrosis.

According to the screening method of the present invention, a test substance is first brought into contact with a biological sample containing cells expressing USP1. As used herein, the term "biological sample" is any sample containing USP1-expressing cells obtained from mammals, including humans, and includes, but is not limited to, tissues, organs, cells, or cell cultures. Specifically, the biological sample includes hepatocytes or a culture thereof.

The term "test substance" used while referring to the screening method of the present invention refers to an unknown substance that is used in screening to examine whether or not it affects the activity or expression level of USP1 at the gene or protein level. Examples of the test substance include, but are not limited to, compounds, nucleotides, antibodies, antisense-RNA, small interference RNA (siRNA), and natural product extracts. Subsequently, the expression level or activity of USP1 in the biological sample treated with the test substance is measured. The measurement of the expression level may be performed by various immunoassay methods known in the art or by various gene detection methods using specifically designed primers or probes targeting a gene whose sequence is known. As a result of the measurement, when the expression level or activity of USP1 decreased, the test substance may be determined as a composition for inhibiting hepatic fibrosis.

As used herein, the term "decrease in expression level or activity" means that the expression level or unique *in vivo* function of USP1 decreases to the extent that liver function is improved to a measurable level by significant inhibition of tissue fibrosis induced by USP1. A decrease in the activity includes not only a simple decrease in function but also an ultimate decrease in the activity due to a decrease in stability. Specifically, the term "decrease in expression level or activity" may mean a state in which the activity or expression level decreased by at least 20%, more specifically at least 40%, even more specifically at least 60%, compared to the control group.

In accordance with yet another aspect of the present invention, the present invention provides a composition for use in a method of preventing, treating or ameliorating a liver disease selected from the group consisting of steatohepatitis, hepatic fibrosis and cirrhosis, the method comprising a step of administering to a subject a composition comprising a compound represented by the following Formula 1 or a food-acceptable salt thereof as an active ingredient.

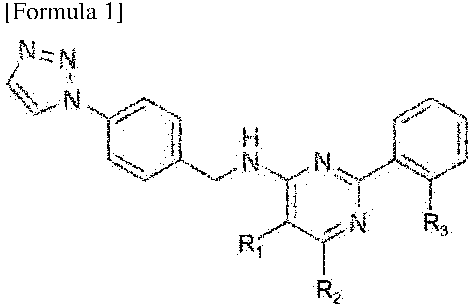

Since the compound of Formula 1 used in the present invention and the liver disease to be ameliorated using the compound have already been described above, description thereof will be omitted to avoid excessive overlapping.

### Advantageous Effects

The features and advantages of the present invention are summarized as follows:
(a) The present invention provides a pharmaceutical composition or a functional food composition for preventing or treating a liver disease selected from the group consisting of steatohepatitis, hepatic fibrosis and cirrhosis.
(b) The triazole derivative compound discovered in the present invention reduces collagen accumulation in liver tissue and significantly inhibits the expression of inflammatory factors. Thus, the triazole derivative compound effectively blocks the progression of a series of severe liver diseases, from excessive fibrogenesis in liver tissue through tissue hardening (cirrhosis) to a decrease in the number of hepatocytes and loss of liver function, unlike conventional drugs that have only a simple lipid-lowering effect. Accordingly, the triazole derivative compound may be useful as a composition for fundamental treatment of liver fibrosis and cirrhosis.

### Brief Description of Drawings

FIG. 1 shows the results of analyzing the viability of LX-2 cells treated with 0, 1, 5, 10 and 25 µM of ML-323 and untreated control cells.
FIG. 2 shows the result of measuring the mRNA expression level of the fibrosis-related factor COL1A1 after treating LX-2 cells with ML323.
FIG. 3 shows the results of observing changes in liver size in a fatty liver mouse model after oral administration of ML-323.
FIG. 4 shows the results of measuring the AST and ALT levels in serum from a fatty liver mouse model after oral administration of ML-323.
FIG. 5 shows the results of observing changes in the expression of collagen (FIG. 5a) and inflammatory factors (FIG. 5b) in liver tissue from a fatty liver mouse model after oral administration of ML-323.
FIG. 6 shows the results of immunohistochemical staining for collagen, which indicate the fibrosis-ameliorating effect of ML-323 in the liver tissue of a fatty liver mouse model after oral administration of ML-323.
FIG. 7 shows the results of staining liver tissue with each of H&E, F4/80 and α-SMA after administering a vehicle and ML-323 to mice fed a non-alcoholic steatohepatitis-inducing diet for 8 weeks.

### Mode for Invention

Hereinafter, the present invention will be described in more detail with reference to examples. These examples are only for explaining the present invention in more detail.

### Examples

### Experimental Methods

### Cytotoxicity measurement

Human hepatic stellate LX-2 cells were seeded in a 96-well plate with DMEM (Welgene) containing 2% fetal bovine serum (Gibco) and 1% penicillin-streptomycin (Gibco), and cultured in an incubator for 24 hours at 37°C under 5% CO₂. Next, the cells were treated with ML323 at concentrations of 0, 1, 5, 10 and 25 µM. After 48 hours, 10 µl of EZ-Cytox was added to the cell culture medium and reacted for 30 minutes, and the absorbance at 450 nm was measured using a microplate reader.

### Measurement of cell fibrosis

Human hepatic stellate LX-2 cells were seeded in a 6-well plate with DMEM (Welgene) containing 2% fetal bovine serum (Gibco) and 1% penicillin-streptomycin (Gibco), and cultured in an incubator for 24 hours at 37°C under 5% CO₂. Thereafter, the medium was replaced with serum-free DMEM, and the cells were starved for 24 hours. Next, the cells were activated by treatment with TGF-β at a concentration of 2.5 ng/ml, and at the same time, the cells were treated with ML323 at concentrations of 0, 1, 10 and 25 µM. After 24 hours, the cells were harvested and used for gene expression analysis.

### Animal model

Twenty-four 6-week-old C57BL/6 mice were acclimated for 1 week and then divided into two groups. One group (12 mice) was fed a normal diet, and the other group (12 mice) was fed a high-fat diet for 9 weeks. After 9 weeks, each of the normal diet group and the high-fat diet group was subdivided into two groups, and each of vehicle and 50 mg/kg of ML323 was orally administered thereto twice a week for 7 weeks. Normal diet and high-fat diet were continued during oral administration.

### Measurement of liver weight change

After the experimental period was over, mice were sacrificed, and liver tissues were harvested and weighed.

### Measurement of liver function indicators in blood

After the experimental period was over, blood was collected from each mouse, and after 30 minutes, the supernatant (plasma) was separated from the blood by centrifugation at 2,000 rpm for 15 minutes. The separated plasma was analyzed by Seoul Clinical Laboratories (SCL) to measure the levels of AST and ALT, which are indicators of liver function.

### Measurement of expression of hepatic fibrosis inducers

The tissue harvested from each mouse was added to 1 ml of Easy blue (Intron) and then minced using a homogenizer. 200 µl of chloroform was added to and mixed well with the tissue, and the mixture was incubated for 3 minutes and then centrifuged at 13,200 rpm for 15 minutes at 4°C. After centrifugation, only the supernatant was transferred to a fresh tube, and the same amount of isopropyl alcohol was added thereto and mixed well therewith. After incubation for 10 minutes, centrifugation was performed at 13,200 rpm for 10 minutes at 4°C. After removing the supernatant, the RNA pellet was washed with 70% ethanol and centrifuged at 13,200 rpm for 5 minutes at 4°C. After removing the supernatant and drying the RNA pellet for 10 minutes, the RNA pellet was dissolved in RNAase-free water.

cDNA was synthesized from the extracted RNA using ReverTraAce RT master mix (TOYOBO), and then gene expression changes were measured using a real-time PCR system (ABI). The primer sets used are summarized in Table 1 below.

**[Table 1] Primer sequences used in real-time PCR**

| Gene | Direction | Primer sequence |
|---|---|---|
| COL1A1 | Forward | 5'-GCTTCACCTACAGCACCCTT-3' |
| | Reverse | 5'-GTCCGAATTCCTGGTCTGGG- 3' |
| CCL2 | Forward | 5'-TAAAAAACCTGGATCGGAACCAA-3' |
| | Reverse | 5'-GCATTAGCTTCAGATTTACGGGT- 3' |
| TGF-β | Forward | 5'-CCTGCAAGACCATCGACATG-3' |
| | Reverse | 5'-TGTTGTACAAAGCGAGCACC- 3' |
| F4/80 | Forward | 5'-CGTCAGCCGATTTGCTATCT-3' |
| | Reverse | 5'-CGGACTCCGCAAAGTCTAAG- 3' |

### Immunohistochemical analysis

The liver tissue harvested from each mouse was fixed in a 10% formalin solution, and a paraffin block and a slide were prepared using each liver tissue that had been fixed. The degree of fibrosis in liver tissue was measured using Sirius red solution staining and a-SMA immunostaining.

In addition, ten 6-week-old C57BL/6 mice were acclimated for 1 week and then fed CDAA (choline-deficient L-amino-defined) for 8 weeks to induce non-alcoholic fatty liver. Thereafter, the 10 mice were divided into two groups, to which a vehicle and 50 mg/kg of ML-323 were orally administered, respectively, and then liver tissues were stained with H&E, F4/80, and α-SMA.

### Experimental results

### Cytotoxicity measurement (WST assay)

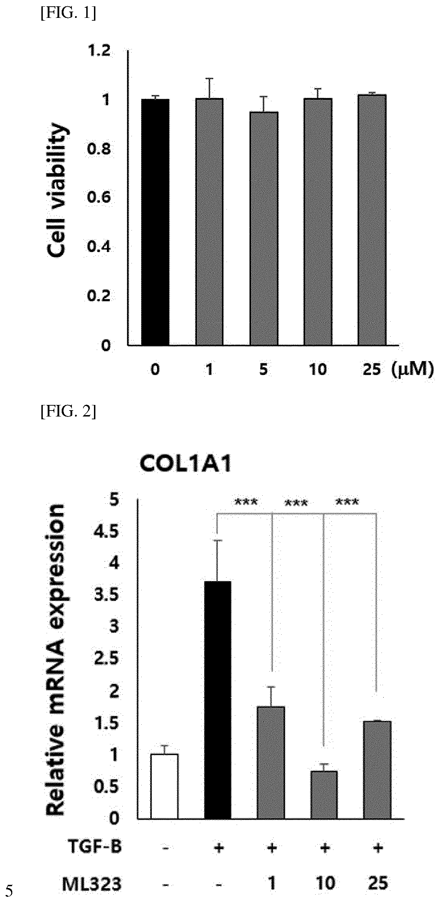

There was no significant difference in viability between cells treated with 0, 1, 5, 10, and 25 µM of ML323 and untreated cells (FIG. 1). Accordingly, it was confirmed that ML323 did not exhibit cytotoxicity at all the treatment concentrations.

### Measurement of cell fibrosis

It was confirmed that the mRNA expression of COL1A1 encoding collagen normally increased in the group treated with TGF-B compared to the control group not treated with TGF-B. It was confirmed that, when cells were treated with TGF-β, the mRNA expression of COL1A1 in the cells treated with ML323 was significantly lower than in the cells not treated with ML323 (FIG. 2). Thereby, it was confirmed that the composition of the present invention efficiently controls hepatic fibrosis in a hepatic fibrosis cell model.

### Measurement of liver weight change

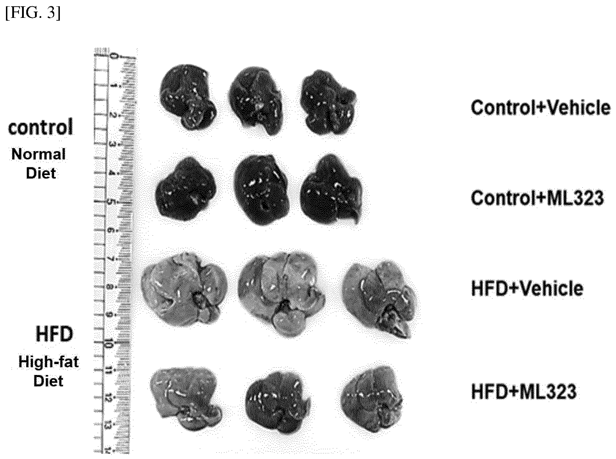

It was confirmed that, in the case of the group fed the normal diet, there was no significant difference in liver weight or size between the ML323-admistered group and the control group, but in the case of the group fed the high-fat diet, the accumulation of fat in liver tissue in the ML323-administered group significantly decreased compared to that in the control group, and the liver weight also decreased in the ML323-administered group (FIG. 3).

### Measurement of liver function indicators in blood

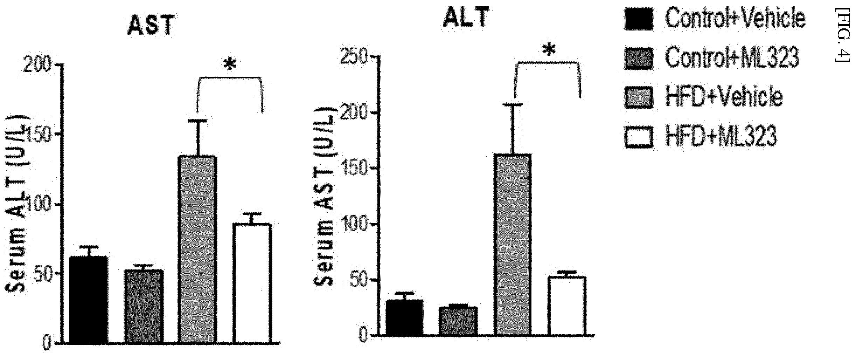

It was confirmed that the levels of AST and ALT in the blood from the ML323-administerd mice in the high-fat diet group significantly decreased by 40% and 70%, respectively, compared to those in the vehicle-administered control group, suggesting that the compound of the present invention significantly restores reduced liver function (FIG. 4).

### Measurement of expression of hepatic fibrosis inducers

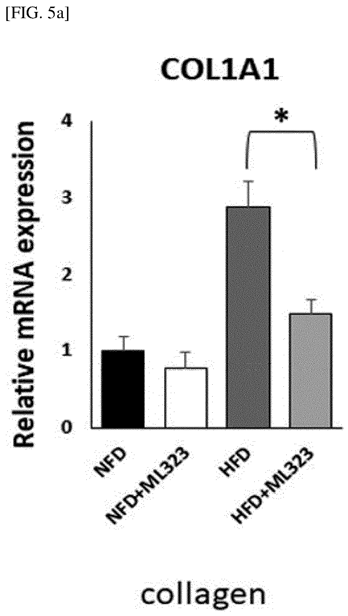

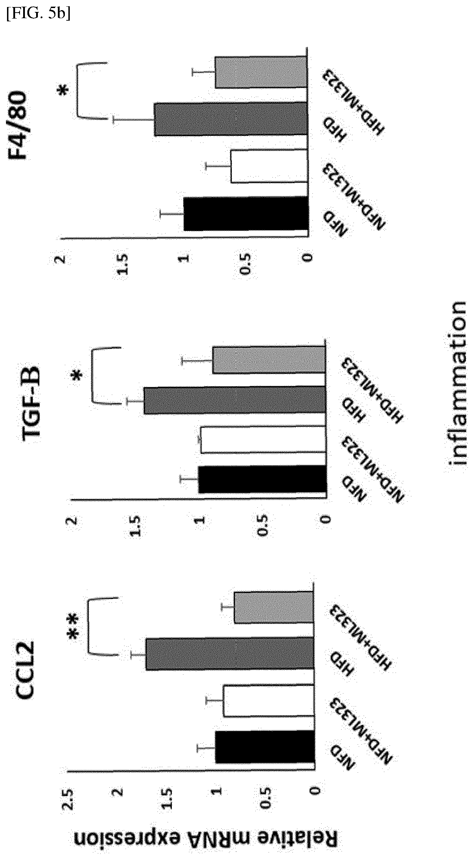

It was observed that the mRNA expression level of the COL1A1 gene encoding type 1 collagen greatly decreased in the liver tissue from the ML323-administered mice in the high-fat diet group (FIG. 5a), and the mRNA expression levels of the inflammation-related genes CCL2, TGF-β and F4/80 also significantly decreased compared to those in the control group (FIG. 5b), suggesting that the composition of the present invention efficiently controls inflammation and fibrosis of liver tissue.

### Immunohistochemical analysis

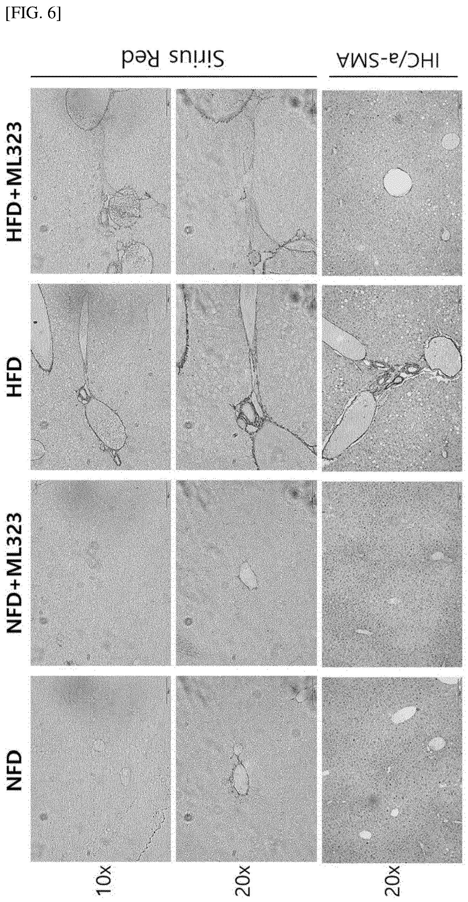

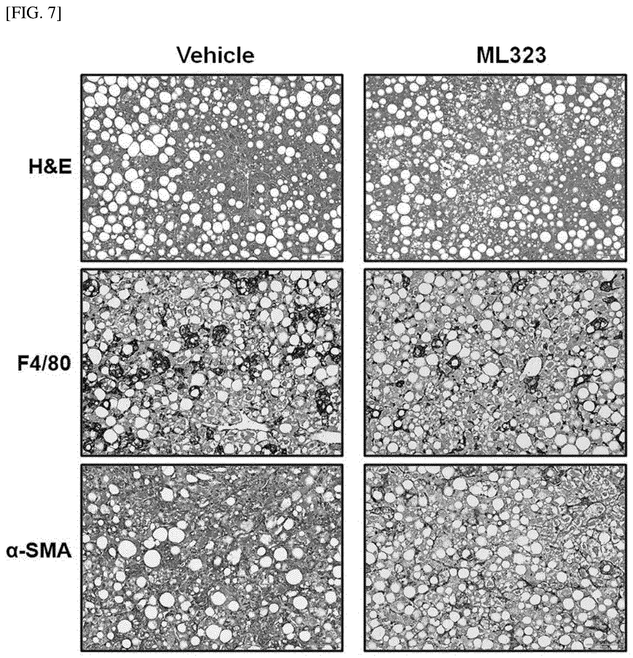

As a result of Sirius red staining and a-SMA immunostaining, it was confirmed that hepatic fibrosis significantly decreased in the liver tissue from the ML323-administered mice (FIG. 6). In addition, as a result of H&E staining, f4/80 immunostaining and a-SMA immunostaining, it was observed that inflammation and hepatic fibrosis significantly decreased in the liver tissue from the ML323-administered group in the CDAA diet group (FIG. 7). From these results, it was confirmed that the composition of the present invention exhibits the effect of histologically ameliorating actual hepatic fibrosis.

Although the present invention has been described in detail with reference to the specific features, it will be apparent to those skilled in the art that this description is only of a preferred embodiment thereof. Thus, the substantial scope of the present invention will be defined by the appended claims.

## Claims

1. A composition for use in a method of preventing or treating a liver disease selected from the group consisting of steatohepatitis, hepatic fibrosis and cirrhosis, comprising a compound represented by the following Formula 1 or a pharmaceutically acceptable salt thereof as an active ingredient: 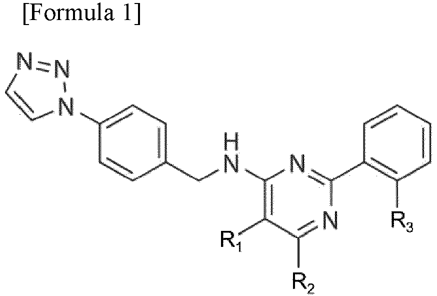 wherein R₁ and R₂ are each independently hydrogen or C₁-C₃ alkyl, R₁ and R₂ are not simultaneously hydrogen, and R₃ is C₂-C₄ alkyl.

2. The composition for use according to claim 1, wherein R₁ is C₁ alkyl, R₂ is hydrogen, and R₃ is C₃ alkyl.

3. The composition for use according to claim 2, wherein R₃ is an isopropyl group.

4. The composition for use according to claim 1, wherein the steatohepatitis is non-alcoholic steatohepatitis.

5. The composition for use according to claim 1, wherein the composition reduces a level of alanine aminotransferase (ALT) or aspartate aminotransferase (AST) in blood.

6. The composition for use according to claim 1, wherein the composition reduces expression of C-C motif chemokine ligand 2 (CCL2), transforming growth factor-β (TGF-β) and F4/80 in liver tissue.

7. A functional food composition for use in a method of ameliorating a liver disease selected from the group consisting of steatohepatitis, hepatic fibrosis and cirrhosis, comprising a compound represented by the following Formula 1 or a food-acceptable salt thereof as an active ingredient: 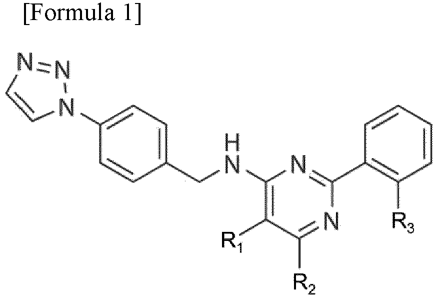 wherein R₁ and R₂ are each independently hydrogen or a C₁-C₃ alkyl, R₁ and R₂ are not simultaneously hydrogen, and R₃ is a C₂-C₄ alkyl.

8. The composition for use according to claim 7, wherein R₁ is C₁ alkyl, R₂ is hydrogen, and R₃ is C₃ alkyl.

9. The composition for use according to claim 8, wherein R₃ is an isopropyl group.

10. A method for screening a composition for inhibiting hepatic fibrosis, comprising:
(1) bringing a test substance into contact with a biological sample containing cells expressing ubiquitin specific protease 1 (USP1); and
(2) measuring an activity or expression level of USP1 in the sample,
wherein, when the activity or expression level of USP1 decreased, the test substance is determined as a composition for inhibiting hepatic fibrosis.

11. The method according to claim 10, wherein the biological sample contains hepatocytes or a culture thereof.

## Patentansprüche

1. Zusammensetzung zur Verwendung in einem Verfahren zum Verhindern oder Behandeln einer Lebererkrankung, ausgewählt aus der Gruppe, bestehend aus Steatohepatitis, Leberfibrose und Leberzirrhose, wobei die Zusammensetzung eine durch die nachstehende Formel 1 repräsentierte Verbindung oder ein pharmazeutisch verträgliches Salz davon als einen aktiven Bestandteil umfasst: 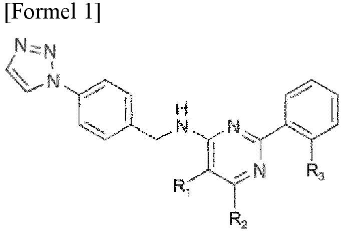 worin R₁ und R₂ jeweils unabhängig Wasserstoffatom oder C₁-C₃-Alkylgruppe sind, R₁ und R₂ nicht gleichzeitig Wasserstoffatom sind, und R₃ eine C₂-C₄-Alkylgruppe ist.

2. Zusammensetzung zur Verwendung gemäß Anspruch 1, wobei R₁ eine C₁-Alkylgruppe ist, R₂ ein Wasserstoffatom ist, und R₃ eine C₃-Alkylgruppe ist.

3. Zusammensetzung zur Verwendung gemäß Anspruch 2, wobei R₃ eine Isopropylgruppe ist.

4. Zusammensetzung zur Verwendung gemäß Anspruch 1, wobei die Steatohepatitis nicht-alkoholische Steatohepatitis ist.

5. Zusammensetzung zur Verwendung gemäß Anspruch 1, wobei die Zusammensetzung einen Spiegel an Alanin-Aminotransferase (ALT) oder Aspartat-Aminotransferase (AST) im Blut verringert.

6. Zusammensetzung zur Verwendung gemäß Anspruch 1, wobei die Zusammensetzung die Expression von C-C-Motiv-Chemokin-Ligand 2 (CCL2), transformierendem Wachstumsfaktor β (TGF-β) und F4/80 im Lebergewebe verringert.

7. Funktionelle Nahrungszusammensetzung zur Verwendung in einem Verfahren zum Verbessern einer Lebererkrankung, ausgewählt aus der Gruppe, bestehend aus Steatohepatitis, Leberfibrose und Leberzirrhose, wobei die Zusammensetzung eine durch die nachstehende Formel 1 repräsentierte Verbindung oder ein nahrungsverträgliches Salz davon als einen aktiven Bestandteil umfasst: 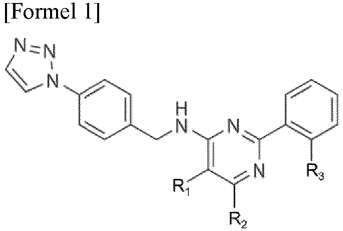 worin R₁ und R₂ jeweils unabhängig Wasserstoffatom oder eine C₁-C₃-Alkylgruppe sind, R₁ und R₂ nicht gleichzeitig Wasserstoffatom sind, und R₃ eine C₂-C₄-Alkylgruppe ist.

8. Zusammensetzung zur Verwendung gemäß Anspruch 7, wobei R₁ eine C₁-Alkylgruppe ist, R₂ ein Wasserstoffatom ist, und R₃ eine C₃-Alkylgruppe ist.

9. Zusammensetzung zur Verwendung gemäß Anspruch 8, wobei R₃ eine Isopropylgruppe ist.

10. Verfahren zum Screenen einer Zusammensetzung auf Hemmung von Leberfibrose, wobei das Verfahren umfasst:
(1) In-Kontakt-Bringen einer Testsubstanz mit einer biologischen Probe, die Zellen enthält, die Ubiquitin-spezifische Protease 1 (USP1) exprimieren; und
(2) Messen eines Aktivitäts- oder Expressionsspiegels von USP1 in der Probe,
wobei, wenn der Aktivitäts- oder Expressionsspiegel von USP1 vermindert ist, bestimmt wird, dass die Testsubstanz eine Zusammensetzung zum Hemmen von Leberfibrose ist.

11. Verfahren gemäß Anspruch 10, wobei die biologische Probe Hepatozyten oder eine Kultur davon enthält.

## Revendications

1. Composition destinée à être utilisée dans un procédé de prévention ou de traitement d'une maladie du foie choisie dans le groupe constitué par la stéatohépatite, la fibrose hépatique et la cirrhose, comprenant un composé représenté par la Formule 1 suivante ou un sel acceptable pharmaceutiquement de celui-ci en tant qu'ingrédient actif : 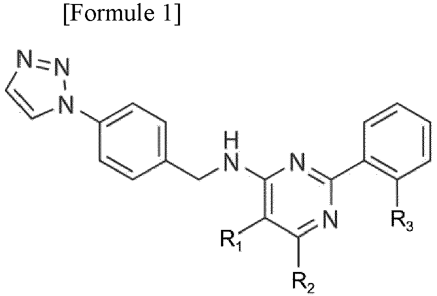 dans laquelle R₁ et R₂ sont chacun indépendamment un hydrogène ou un alkyle en C₁-C₃, R₁ et R₂ ne sont pas simultanément un hydrogène, et R₃ est un alkyle en C₂-C₄.

2. Composition destinée à être utilisée selon la revendication 1, dans laquelle R₁ est un alkyle en C₁, R₂ est un hydrogène et R₃ est un alkyle en C₃.

3. Composition destinée à être utilisée selon la revendication 2, dans laquelle R₃ est un groupe isopropyle.

4. Composition destinée à être utilisée selon la revendication 1, ladite stéatohépatite étant une stéatohépatite non alcoolique.

5. Composition destinée à être utilisée selon la revendication 1, ladite composition réduisant un taux d'alanine aminotransférase (ALAT) ou d'aspartate aminotransférase (ASAT) dans le sang.

6. Composition destinée à être utilisée selon la revendication 1, ladite composition réduisant l'expression de la chimiokine ligand 2 à motif C-C (CCL2), transformant le facteur de croissance-β (TGF-β) et F4/80 dans le tissu hépatique.

7. Composition alimentaire fonctionnelle destinée à être utilisée dans un procédé d'amélioration d'une maladie du foie choisie dans le groupe constitué par la stéatohépatite, la fibrose hépatique et la cirrhose, comprenant un composé représenté par la Formule 1 suivante ou un sel acceptable pour les aliments de celui-ci en tant qu'ingrédient actif : 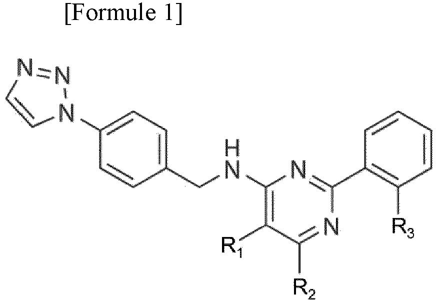 dans laquelle R₁ et R₂ sont chacun indépendamment un hydrogène ou un alkyle en C₁-C₃, R₁ et R₂ ne sont pas simultanément un hydrogène, et R₃ est un alkyle en C₂-C₄.

8. Composition destinée à être utilisée selon la revendication 7, dans laquelle R₁ est un alkyle en C₁, R₂ est un hydrogène et R₃ est un alkyle en C₃.

9. Composition destinée à être utilisée selon la revendication 8, dans laquelle R₃ est un groupe isopropyle.

10. Procédé permettant la sélection d'une composition destinée à inhiber la fibrose hépatique, comprenant :
(1) la mise en contact d'une substance d'essai avec un échantillon biologique contenant des cellules exprimant la protéase 1 spécifique de l'ubiquitine (USP1) ; et
(2) la mesure d'un niveau d'activité ou d'expression d'USP1 dans l'échantillon,
dans lequel lorsque le niveau d'activité ou d'expression d'USP1 diminue, la substance d'essai est déterminée comme une composition permettant d'inhiber la fibrose hépatique.

11. Procédé selon la revendication 10, dans lequel l'échantillon biologique contient des hépatocytes ou une culture de ceux-ci.
